# EUROPEAN PATENT APPLICATION

(11) **EP 1 862 168 A1**
(43) Date of publication of application: **05.12.2007**
(21) Application number: 06728680.7
(22) Date of filing: 07.03.2006
(51) Int. Cl.: A61K 31/695, A61P 1/16, A61P 35/00

(54) **METHOD OF TREATING LIVER CANCER**

(30) Priority: 08.03.2005 US 659078 P
(71) Applicant: TAIHO PHARMACEUTICAL COMPANY LIMITED, Chiyoda-ku, Tokyo 101-8444 (JP)
(72) Inventor: ARIMA, Takashi, hiki-cho, Chiyoda-ku, Tokyo, 1010054 (JP)
(74) Representative: Blum, Erwin
(86) International application number: PCT/JP2006/304311
(87) International publication number: WO 2006/095704

(57) **Abstract**

A method for therapeutic treatment of hepatic cancer, which comprises the step of administering 10 to 30 mg per day of 4-[3,5-bis(trimethylsilyl)benzamido]-benzoic acid or a physiologically acceptable salt thereof to a patient with hepatic cancer.

## Description

### Field of the Invention

The present invention relates to a method for treatment of hepatic cancer which uses 4-[3,5-bis(trimethylsilyl)benzamido]benzoic acid at a low dose.

### Background Art

Hepatic cancer is highly malignant, and one million patients die from the disease every year, which number of the patients is in the fifth place [EI-Serag, H. B., and Mason, A. C., Rising incidence of hepatocellular carcinoma in the United States., N. Engl. J. Med., 340, pp.745-750, 1999; Taylor-Robinson, S. D., Foster, G. R., Arora, S., Hargreaves, S., and Thomas, H. C., Increase in primary liver cancer in the UK, 1979-94 Lancet, 350, pp.1142-1143, 1997].

It is considered that the onset of hepatic cancer involves generation of plural cancer cells in different positions just or almost simultaneously due to exposure of the liver with hepatic viruses or alcohol [Slaughter DP, Southwick HW, Smejkal W., "Field cancerization" in oral stratified squamous epithelium; Clinical implications of multicentric origin., Cancer, 6, pp.963-968, 1953]. In our country, a major cause of the generation of hepatic cancer is considered to be hepatitis C virus infection. A possibility of recurrence of the cancer is high, because hepatitis C viruses cannot be completely eliminated even if cancer cells are temporarily removed or become extirpated. A rate of the recurrence was reported to be 16 to 29% from 1990 to 1997 [Hepatic Cancer, "I. Change of Number of Patients", "3. Method for Calculation of Number of Patients, Tendency of Cancer Patients and Actual Condition According to Therapeutic Method", Medical Research Co., Ltd., pp.20-30, 2001].

As methods for treatment of hepatic cancer, a surgical hepatic resection for a visible lesion, a radio-wave thermocauterization therapy, an ethanol infusion therapy, a microwave coagulation-necrosis therapy and the like are available. However, these treatments are sometimes not sufficiently effective, and therefore, development of chemotherapy as a systemic treatment has been desired.

As a chemotherapy, an intra-arterial injection therapy using CDDP, ADM, or 5-FU has recently been employed to patients with advanced cancer, to whom the above-mentioned therapy or transcatheter arterial embolization is not applicable, and to patients with recurrence after these treatments. However, as for the intra-arterial injection therapy using a combination of lipiodol as an oily contrast medium and the aforementioned medicament, efficacy was reported to be 13.0% of complete response and 30.0% effective, and as for chemotherapies excluding a combination of the transcatheter arterial embolization and lipiodol, efficacy was reported to be as high as 2.5% of complete response and 3.1% of effective, which clinical results are not satisfactory. Further, the aforementioned combination therapies have a problem such as occurrence of complications such as an ulcer [Hisakazu Tanigawa, "Topical Injection Therapy and Intra-arterial Injection Therapy", Clinical Therapy of Cancer, 40, pp.1490-1497, 1994].

As explained above, a standard method for therapeutic treatment of hepatic cancer has not yet been available which constantly guarantees a certain level of clinical results. Especially, intra-arterial injection has problems from viewpoints of techniques and patient's burden, and therefore, development of a chemotherapy in place of this therapy has been desired. Further, since hepatic cancer has a high resistance to a chemotherapy, even stabilization of a cancer, i.e., a therapy to prevent proliferation of a tumor for prolongation of life, is difficult to be achieved. Therefore, development of a chemotherapy has been strongly desired which enables conservative treatment of a tumor.

4-[3,5-Bis(trimethylsilyl)benzamido]benzoic acid (hereinafter in the specification, this compound may be referred to as "TAC-101") is a synthetic retinoid represented by the following chemical formula (1), and the compound is known to be useful as an agent for cancer treatment, a differentiation-inducing agent of a cancer cell, a cancer metastasis inhibitor, a therapeutic agent for vascular diseases, or an agent for treatment of cardiac hypertrophy (Japanese Patent Unexamined Publication (KOKAI) No. 2-247185, pamphlet of International-Publication WO 96/32101, pamphlet of International-Publication WO 03/089005 and the like). 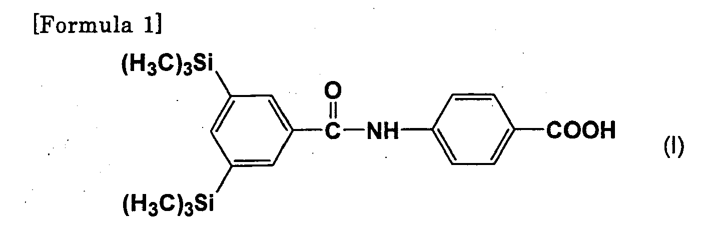 TAC-101 exhibits the antitumor effect by binding selectively to intranuclear retinoic acid receptor α (RAR-α) thereby activating transcription through the receptor. At present, this compound has been under clinical development as an orally available antitumor agent.

In pharmacological experiments using an animal model of hepatic cancer, antitumor effects of TAC-101 were demonstrated [Murakami K, Matsuura T, Sano M, et al., 4-[3,5-Bis(trimethylsilyl)benzamido]benzoic acid (TAC-101) inhibits the intrahepatic spread of hepatocellular carcinoma and prolongs the life-span of tumor-bearing animals., Clin. Exp. Metastasis. 16, pp.633-643, 1998; Murakami K, Wierzba K, Sano M, et al. TAC-101, a benzoic acid derivative, inhibits liver metastasis of human gastrointestinal cancer and prolongs the life-span., Clin. Exp. Metastasis., 16, pp.323-331, 1998]. However, clinical effect of TAC-101 for patients with hepatic cancer has not yet been known so far.

In the United States of America, clinical phase I trials and clinical phases I/II trials have been conducted where TAC-101 is administered at a high dose in a range of 60 to 80 mg/day to patients with lung cancer, and these trials have revealed that the drug exhibits tumor reducing effects (PR and CR). However, it has also been found that the administration of the drug frequently induces deep vein thrombosis (DVT), and due to the appearance of the side effect as being dose-limiting toxicity, the dose fails to sufficiently achieve the clinical purpose of therapeutic treatment of the cancer [ Naiyer A. et al. Initial Clinical Trial of Oral TAC-101, a Novel Retinoic Acid Receptor-Alpha Selective Retinoid, in Patients With Advanced Cancer,, Journal of Clinical Oncology, 20, pp.3522-3532, 2002].

### Disclosure of the Invention

### Object to be Achieved by the Invention

An object of the present invention is to provide a novel and effective chemotherapeutic method for treatment of hepatic cancer.

### Means to Achieve the Object

In order to achieve the foregoing object, the inventors of the present invention conducted various researches on dosage schedules for patients with hepatic cancer using TAC-101. As a result, the inventors found surprisingly that a low dose of 10 to 30 mg per day sufficiently achieved satisfactory therapeutic effect on hepatic cancer to almost completely prevent advance of hepatic cancer, and the dose gave almost no generation of deep vein thrombosis so as to enable extremely safe chemotherapeutic treatment of hepatic cancer. The present invention was achieved on the basis of these findings.

The present invention thus provides a method for therapeutic treatment of hepatic cancer, which comprises the step of administering 10 to 30 mg per day of 4-[3,5-bis(trimethylsilyl)benzamido]benzoic acid or a physiologically acceptable salt thereof to a patient with hepatic cancer.
According to preferred embodiments of the aforementioned invention, provided are: the above method wherein the therapeutic treatment of hepatic cancer is arresting therapy of hepatic cancer; the above method wherein the therapeutic treatment of hepatic cancer is conservative therapy of hepatic cancer; and the above method wherein generation of deep vein thrombosis is substantially avoidable in the therapeutic treatment of hepatic cancer.

According to more preferred embodiments of the present invention, provided are: the above method which comprises the step of administering 15 to 25 mg per day of 4-[3,5-bis(trimethylsilyl)benzamido]benzoic acid or a physiologically acceptable salt thereof; the above method which comprises the step of administering 20 mg per day of 4-[3,5-bis(trimethylsilyl)benzamido]benzoic acid or a physiologically acceptable salt thereof the above method wherein the treatment is carried out according to therapeutic schedule comprising continuous daily administration for 1 to 4 weeks, followed by drug withdrawal for 1 to 3 weeks; the above method wherein the treatment is carried out according to therapeutic schedule comprising continuous daily administration for 2 weeks, followed by drug withdrawal for 1 week; the above method wherein either of the aforementioned therapeutic schedules is repeated at least two times; the above method wherein the hepatic cancer is advanced hepatocellular carcinoma; the above method wherein the hepatic cancer is primary hepatic cancer; and the above method wherein the treatment is carried out together with a therapy selected from the group consisting of a surgical hepatic resection, a radio-wave thermocauterization therapy, an ethanol infusion therapy, and a microwave coagulation-necrosis therapy.

From another aspect of the present invention, a medicament for therapeutic treatment of hepatic cancer is provided which comprises 10 to 30 mg of 4-[3,5-bis(trimethylsilyl)benzamido]benzoic acid or a physiologically acceptable salt thereof per single administration unit. Preferably, provided is the above medicament which comprises 15 to 25 mg of 4-[3,5-bis(trimethylsilyl)benzamido]benzoic acid or a physiologically acceptable salt thereof per single administration unit; and more preferably, provided is the above medicament which comprises 20 mg of 4-[3,5-bis(trimethylsilyl)benzamido]benzoic acid or a physiologically acceptable salt thereof per single administration unit.
From further aspect of the present invention, provided is a use of 4-[3,5-bis(trimethylsilyl)benzamido]benzoic acid or a physiologically acceptable salt thereof for manufacture of a medicament for therapeutic treatment of hepatic cancer, wherein said medicament is used for administration of 10 to 30 mg per day of 4-[3,5-bis(trimethylsilyl)benzamido]benzoic acid or a physiologically acceptable salt thereof. According to preferred embodiments, provided are the aforementioned use, wherein the medicament is used for administration of 15 to 25 mg per day of 4-[3,5-bis(trimethylsilyl)benzamido]benzoic acid or a physiologically acceptable salt thereof, and the aforementioned use, wherein the medicament is used for administration of 20 mg per day of 4-[3,5-bis(trimethylsilyl)benzamido]benzoic acid or a physiologically acceptable salt thereof. According to the aforementioned use, said medicament used for oral administration is preferred.

### Best Mode for Carrying out the Invention

The active ingredient TAC-101 used in the therapeutic method of the present invention can be manufactured, for example, according to the method described in the Japanese Patent Unexamined Publication (KOKAI) No. (Hei)2-247185.
The therapeutic method of the present invention is characterized in that TAC-101 as the active ingredient is administered at 10 to 30 mg per day. According to the therapeutic method of the present invention, for example, a unit dosage of a medicament in an appropriate form, which comprises TAC- 10 as the active ingredient, is prepared, and the aforementioned dose is administered by administration of one or more of the dosage units to a patient. Preferably, a medicament comprising 10 to 30 mg of TAC-101 per a single dosage unit is prepared and administered. A route of administration is not particularly limited, and the medicament is administered orally or parenterally. Oral administration is preferred from a viewpoint of convenience for administration.

Examples of the form of the medicament suitable for oral administration include solid preparations such as tablets, coated tablets, pills, powders, granules, and capsules, and non-solid preparations such as solutions, suspensions, and emulsions. In these preparations, examples of the single dosage unit include one tablet for the tablets, one capsule for the capsules, one unit package divided by means of heat seal for the powders or subtilized granules, or one container or one vial for non-solid preparations, which forms are well known to one of ordinary skill in the art. The aforementioned preparations can be packaged so that the daily dose of 10 to 30 mg can be administered once or dividedly 2 to 4 times a day. A method for the package is not limited so far that the method is known as a commonly used packaging method in the field. For example, tablets can be packaged in a package material for shielding moisture and oxygen.

For manufacture of these preparations, one or more pharmaceutical additives such as pharmaceutically acceptable carrier may be used. The aforementioned preparations can be manufactured according to commonly used methods for formulation ordinarily known in the art.
As carriers for formulation in a form of tablets, excipients such as lactose, sucrose, sodium chloride, glucose, urea, starch, calcium carbonate, kaoline, crystalline cellulose, and silicic acid; binders such as water, ethanol, propanol, cornstarch, simple syrup, glucose solution, starch solution, gelatin solution, carboxymethylcellulose, shellac, methylcellulose, hydroxypropylcellulose, hydroxypropylmethylcellulose, potassium phosphate, and polyvinylpyrrolidone; disintegrators such as dried starch, sodium alginate, agar pulveratum, laminaran powder, sodium bicarbonate, calcium carbonate, polyoxyethylene sorbitan fatty acid esters, sodium lauryl sulfate, stearic acid monoglyceride, starch, and lactose; disintegration suppressing agent such as sucrose, stearic acid, cacao butter, and hydrogenated oil; absorption enhancers such as a quaternary ammonium salt and sodium lauryl sulfate; moisturizers such as glycerol and starch; adsorbents such as starch, lactose, kaoline, bentonite, and colloidal silicic acid; and lubricants such as purified talc, a stearic acid salt, boric acid powder, and polyethylene glycol. Tablets may optionally be prepared as tablets with an ordinary coating such as sugar-coated tablets, gelatin-protective tablets, enteric-coated tablets, film coated tablets, double layered tablets, and multilayered tablets and the like may be used.

As carriers for formulation in a form of pills, excipients such as glucose, lactose, starch, cacao oil, hardened vegetable oil, kaoline, and talc; binders such as gummi arabicum pulveratum, powdered tragacanth, gelatin, and ethanol; disintegrating agents such as laminaran and agar and the like may be used.
Capsules may be manufactured according to ordinary methods by mixing the aforementioned ingredient with the above exemplified various carriers and then filling the mixture in hard gelatin capsule, soft capsules or the like.
When oral liquid preparations are desired, orally administrable solutions, syrups, elixirs and the like may be manufactured by an ordinary method by using flavor-corrective agents, buffering agents, stabilizers, smell-corrective agents and the like. In the above preparations, examples of the flavor-corrective agents include sucrose, bitter orange peel, citric acid, tartaric acid and the like, examples of the buffering agents include sodium citrate and the like, and examples of the stabilizers include tragacanth, gum arabic, gelatin and the like.
The aforementioned preparations may optionally be added with colorants, preservative, perfumes, flavoring agents, sweetening agents and the like, or other drugs.

According to the therapeutic method of the present invention, TAC-101 may be administered once a day or dividedly 2 to 4 times a day so as to achieve the dose of 10 to 30 mg per day. The dose may be appropriately chosen depending on a method of administration, conditions of a patient such as age, sexuality and the like, and severity of the disease and the like. More preferred daily dose may be 15 to 25 mg per day, and most preferred dose may be 20 mg per day. On the basis of the selection of the aforementioned dose, generation of deep vein thrombosis can be substantially completely prevented, while a high therapeutic effect on hepatic cancer can be maintained. Further, on the basis of the selection of the aforementioned dose, progress of hepatic cancer can be substantially prevented, thereby conservative therapy of hepatic cancer can be achieved while adverse reactions are avoided.

As therapeutic schedules, a therapeutic schedule is preferred wherein continuous daily administration for 1 to 4 weeks is carried out, and then a period of drug withdrawal for 1 to 3 weeks is provided. A therapeutic schedule is more preferred wherein continuous daily administration for 2 weeks is carried out, and then a period of drug withdrawal for 1 week is provided. At least two times repetition of either of the aforementioned therapeutic schedules is more preferred, and at least four times repetition is most preferred.
Types of hepatic cancer as the target of the therapeutic method of the present invention are not particularly limited. For example, the therapy is applicable to any of hepatocellular carcinoma, cholangiocellular carcinoma, hepatocyte blastoma, hepatocytes and cholangiocellular mixed cancer, anaplastic carcinoma bile duct cystadenocarcinoma, carcinoid tumor or the like. A preferred applicable target is hepatocellular carcinoma. Hepatic cancer is basically classified into primary hepatic cancer and metastatic hepatic cancer. The therapeutic method of the present invention is applicable to any of these cancers. A preferred applicable target is primary hepatic cancer. In particular, the method of the present invention may be applied as an excellent therapeutic method to advanced hepatocellular carcinoma.

### Examples

The present invention will be more specifically explained by way of examples. However, the scope of the present invention is not limited to these examples.

| Example 1: Tablets | |
|---|---|
| TAC-101 | 20 mg |
| Starch | 100 mg |
| Magnesium stearate | 15 mg |
| Lactose | 45 mg |
| Total | 180 mg |

On the basis of the above ratios of the ingredients, tablets of 180 mg per tablet were manufactured according to an ordinary method.

| Example 2: Granules | |
|---|---|
| TAC-101 | 15 mg |
| Lactose | 340 mg |
| Cornstarch | 450 mg |
| Hydroxypropylmethylcellulose | 10 mg |
| Total | 820 mg |

On the basis of the above ratios of the ingredients, granules were prepared according to an ordinary method.

| Example 3: Capsule | |
|---|---|
| TAC-101 | 10 mg |
| L-Hydroxypropylcellulose | 20 mg |
| Hydroxypropylmethylcellulose | 5 mg |
| Magnesium Stearate | 3 mg |
| Total | 48 mg |

On the basis of the above ratios of the ingredients, capsules were prepared according to an ordinary method.

### Example 4: Clinical Study

### <Method>

To patients with advanced hepatocellular carcinoma, two courses of treatment with TAC-101 were applied, which each course comprised continuous daily oral administration for 14 days followed by drug withdrawal for 7 days. An average age of the patients was 65 (ranging from 30 to 85). 22 patients received prior therapies, which details are as follows: 18 patients (55%) with chemotherapy; 10 patients (30%) with surgery; 4 patients (12%) with radiotherapy; 1 patient (3%) with immunotherapy; and 2 patients (6%) with other therapy. Administrations were continued for patients without dose-limiting toxicities (DLT) and without aggravation of the cancer. According to the Simon's "optimal" design [Simon, R., "Optimal two-stage designs for phase 2 clinical trials", Control Clin. Trials, 10, pp. 1-10, 1989], a recommended dose was determined in Phase I part, and then Phase II part was started without delay. Patients administered with the recommended dose in Phase I part were subjected to data analysis under assumption as Phase II part patients.

Phase I part was started at a dose of 40 mg/day of TAC-101. DLT was observed for two patients among five, and accordingly, the dose was reduced to 20 mg/day for administration to seven patients.
In Phase II part, additional 21 patients were treated at 20 mg/day. All patients administered with TAC-101 were subjected to evaluation for safety, and patients administered at least for 28 days (i.e., 2 courses) were subjected to evaluation for efficacy. Efficacy judgment was conducted according to the WHO criteria every 6 weeks [WHO handbook for reporting results of cancer treatment. Geneva (Switzerland): World Health Organization Offset Publication No. 48; 1979]. Specifically, after determination of the measurable lesions in each patient, the response was classified as complete response (CR), partial response (PR), minor response (MR), stabilization of the disease (SD), or progression of the disease (PD) for the efficacy evaluation. In the above trial, "CR" means disappearance of all of the lesions observed for four weeks or more; "PR" means 50% or more reduction of the product of the longest diameter and the perpendicular shortest diameter of all of the observable lesions relative to the baseline, and the condition was maintained for 4 weeks or more; "MR" means 25 to 50% reduction of the product of the longest diameter and the perpendicular shortest diameter of all of the observable lesions relative to the baseline, or reduction of an increased value of α-fetoprotein (AFP) without proliferation of the tumor; "SD" means reduction of the tumor is not as high as that of MR, and proliferation of the tumor is not as high as that of PD; and "PD" means 25% or more increase of the minimum tumor size at the start of the therapy, or appearance of new legions.

### <Results>

Among 5 patients treated at 40 mg/day in Phase I part, 2 patients were observed to develop DLT. One patient developed arthralgias, myalgias, exfoliative dermatitis, and venous thrombotic event, and the other patient developed fatigue, dermatitis, and vena cava thrombosis. Among 7 patients treated at 20 mg/day, only one patient was observed to develop DLT (pancreatitis and fatigue), and accordingly, 20 mg/day was determined as maximum tolerance dose (MTD). Among 28 patients treated at 20 mg/day, treatments including 2 courses or more were applicable to 21 patients, and these patients were subjected to the evaluation for efficacy. A median treatment during the administration period was 2 courses of treatment (ranging from 1 to 14). Although PR or CR case was not observed, four or more courses of treatments were applicable to 9 patients for whom progress of the hepatic cancer was significantly arrested.

For all of the 21 patients administered at 20 mg/day, time to treatment failure (TTF) was 6.6 weeks (ranging from 1.3 to 48). Among them, TTFs were 12 weeks or longer for 9 patients, for whom progress of the hepatic cancer was significantly arrested for a long period of time and no deterioration was observed (9/21, 42%). In the above treatments, although the therapies were terminated for one patient because of the onset of grade 4 pulmonary embolism (PE), for one patient with the onset of grade 3 venous thrombosis embolism (VTE), and one patient with grade 3 increase of aspartate aminotransferase (AST), most of patients only suffered from slight side effects approximately in grade 2, which details are: fatigue (9 patients/32%), myalgias and increased triglycerides (each 8 patients/28.6%), dermatitis (6 patients/21.4%), AST increase, alanine aminotransferase (ALT), and nausea (each 4 patients/14.3%), and anorexia (3 patients/10.7%).

### <Conclusion>

From the above results, it was revealed that the therapeutic method comprising administration of TAC-101 1 at 20 mg/day, particularly administration for 2 weeks at an interval of every 3 weeks, was highly acceptable to patients with progressive hepatic cancer. In addition, long-term administration was successfully applicable to some of the patients, and these patients were observed to give remarkable stabilization of the tumor for a prolonged period of time.

### Industrial Applicability

According to the therapeutic method of the present invention, sufficient therapeutic effect can be achieved on hepatic cancer, and progress of hepatic cancer can be almost completely arrested. In the therapeutic method of the present invention, almost no generation of deep vein thrombosis is observed, thereby extremely safe chemotherapy of hepatic cancer is achieved.

## Claims

1. A method for therapeutic treatment of hepatic cancer, which comprises the step of administering 10 to 30 mg per day of 4-[3,5-bis(trimethylsilyl)benzamido]benzoic acid or a physiologically acceptable salt thereof to a patient with hepatic cancer.

2. The method according to claim 1, wherein the therapeutic treatment of hepatic cancer is arresting therapy of hepatic cancer.

3. The method according to claim 1, wherein the therapeutic treatment of hepatic cancer is conservative therapy of hepatic cancer.

4. The method according to any one of claims 1 to 3 wherein generation of deep vein thrombosis is substantially avoidable in the therapeutic treatment of hepatic cancer.

5. The method according to any one of claims 1 to 4, which comprises the step of administering 15 to 25 mg per day of 4-[3,5-bis(trimethylsilyl)benzamido]benzoic acid or a physiologically acceptable salt thereof.

6. The method according to any one of claims 1 to 4, which comprises the step of administering 20 mg per day of 4-[3,5-bis(trimethylsilyl)benzamido]benzoic acid or a physiologically acceptable salt thereof.

7. The method according to any one of claims 1 to 6, wherein the treatment is carried out according to therapeutic schedule wherein continuous daily administration for 1 to 4 weeks is carried out, and then a period of drug withdrawal for 1 to 3 weeks is provided.

8. The method according to any one of claims 1 to 6, wherein the treatment is carried out according to therapeutic schedule wherein continuous daily administration for 2 weeks is carried out, and then a period of drug withdrawal for 1 week is provided.

9. The method according to claim 7 or claim 8, wherein either of the aforementioned therapeutic schedules is repeated at least two times.

10. The method according to any one of claims 1 to 9, wherein the hepatic cancer is advanced hepatocellular carcinoma.

11. The method according to any one of claims 1 to 10, wherein the hepatic cancer is primary hepatic cancer.

12. A medicament for therapeutic treatment of hepatic cancer which comprises 10 to 30 mg of 4-[3,5-bis(trimethylsilyl)benzamido]benzoic acid or a physiologically acceptable salt thereof per single administration unit.

13. The medicament according to claim 12, which is an orally available preparation.

14. The medicament according to claim 13, which is a tablet, a capsule, or a powder.

15. Use of 4-[3,5-bis(trimethylsilyl)benzamido]benzoic acid or a physiologically acceptable salt thereof for manufacture of a medicament for therapeutic treatment of hepatic cancer, wherein the medicament is used for administration of 10 to 30 mg of 4-[3,5-bis(trimethylsilyl)benzamido]benzoic acid or a physiologically acceptable salt thereof per day.
